# EUROPEAN PATENT APPLICATION

(11) **EP 2 478 902 A1**
(43) Date of publication of application: **25.07.2012**
(21) Application number: 10816760.2
(22) Date of filing: 13.09.2010
(51) Int. Cl.: A61K 31/365, A61K 31/205

(54) **PHARMACEUTICAL COMPOSITION FOR REDUCING WEIGHT AND METHOD FOR THE PRODUCTION THEREOF**

(30) Priority: 18.09.2009 MX 2009009971
(71) Applicant: World-Trade Import-Export Wtie, AG, 6302 Zug (CH)
(72) Inventor: GARCÍA ARMENTA, María Elena, 45020 Zapopan (MX); SANTOS MURILLO, Josefina, Jalisco (MX); ÁLVAREZ OCHOA, Víctor Guillermo, Jalisco (MX)
(74) Representative: Carpintero Lopez, Francisco
(86) International application number: PCT/IB2010/002270
(87) International publication number: WO 2011/033356

(57) **Abstract**

A pharmaceutical composition is described for reducing weight, which comprises a mixture of Orlistat and L-carnitine, as active ingredients and a pharmaceutically acceptable vehicle. Also described is a method for producing said composition, in which the Orlistat undergoes moistening and moist granulation, which offsets problems of sticking or poor fluidity in the case or this compound.

## Description

### FIELD OF INVENTION

This invention relates to the techniques used in the pharmaceutical industry and, more in particular, relates to a pharmaceutical composition for reducing weight that comprises as active ingredients Orlistat and L-carnitine, as well as the method for obtaining it.

### BACKGROUND OF THE INVENTION

Obesity has become one of the greatest threats for the population due to its growing incidence and its capacity to cause chronic diseases in almost all body systems. In the past fifteen years, advances have been made in the understanding of the disease that surpass those reached in the previous hundred years, and this has created a rational base for new therapies that can prevent or reverse obesity.

Due to its significance and impact, obesity is considered to be a public health problem in Mexico, as reported in the national health survey of the year 2000, that identifies that a high prevalence of overweight and obesity exists from early ages, as adolescents show 29 % of overweight and obesity and in adults women aged 20-59 years 36.1 % overweight and 28.1 % obesity were obtained and in adult men of the same group 40.9 % overweight and 18.6 % obesity.

Furthermore, obesity and overweight have harmful effects on health on the short and long term in children, including: psychiatric disorders, hypertension, dyslipidaemia, left ventricular hypertrophy, non-alcoholic steatohepatitis, endothelial function disorders, hyperinsulinaemia or insulin resistance, asthma, obstructive sleep apnoea and orthopaedic problems. It is currently known that the longer obesity is present, the higher the mortality and morbidity risks.

Therefore, all possible therapeutic resources must be considered to manage overweight and obesity, from physical exercise to new pharmaceutical products. Exercise per se improves sensitivity to insulin and reduces the risk of diabetes; an adequate diet and additional measures no doubt help achieve the objective of reducing weight with the maximum possible benefit.

In the prior art some compounds and compositions are known to have been used to reduce weight, one of them is N-formyl-L-leucine (1 S)-1-[[(2S, 3S)-3-hexyl-4-oxo-2-oxethanyl]methyl]dodecyl ester), more known as Orlistat of formula (I):

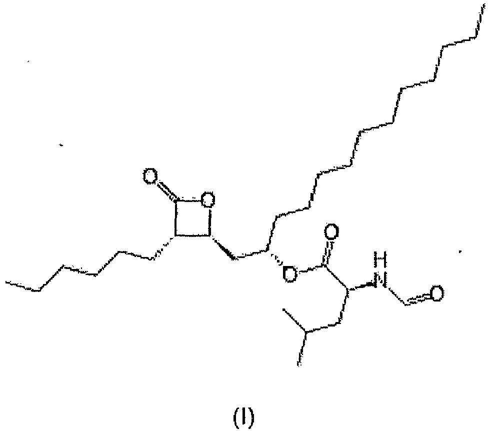

Orlistat, also known as tetrahydrolipstatin, is a drug for treating obesity, and its main function is to prevent absorption of fats from human diet, thus reducing calorie intake. Orlistat acts by inhibiting pancreatic lipase, an enzyme that breaks down triglycerides in the bowel. Without this enzyme, diet triglycerides are not hydrolysed into absorbable fatty acids and are excreted undigested. Their main effect is the local inhibition of lipase in the gastrointestinal tract after taking this compound in an oral dose.

Another compound that has been used to treat overweight and obesity is L-carnitine; of its salts that most commonly used is L-carnitine tartrate (C₁₈H₃₆N₂O₁₂), (R)-Bis [(3-carboxy-2-hydroxypropyl)trimethyl Ammonium] L-tartrate, of formula (II).

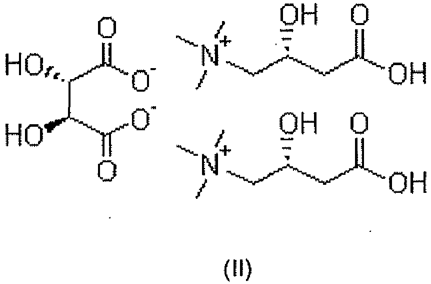

Due to its role in lipid metabolism, L-carnitine is used as coadjuvant in the medical management of weight control in patients with exogenous obesity, due to an excessive and uncontrolled food intake.

In the international patent application PCT/EP02/07812, a composition created for the main purpose of absorbing fat-soluble vitamins is described; this composition comprises an inhibitor of pancreatic lipase, such as Orlistat and L-Carnitine, and each of them is administered independently, i.e., they are never mixed. During the study of this composition, a beneficial side effect was seen in weight reduction. Specifically the composition of this document uses a daily dose of 50-300 mg of Orlistat and 50 to 8,000 mg of L-Carnitine.

In particular, to achieve the weight reduction effect the document mentions a daily dose of 500 to 8,000 mg of L-Carnitine in an adult. In a modality of example, in this document Orlistat was used at a daily dose of 360 mg in three capsules each containing 120 mg, and for L-Carnitine a daily dose of 1,000 mg was used before each of the three foods in an effervescent tablet, which amounted to 3,000 mg a day. However, this document does not mention the quantitative weight loss seen, it only mentions a qualitative loss, but particularly it does not show if amounts below 3,000 mg of L-Carnitine per day achieve weight loss.

From this document it may be concluded that, to achieve weight reduction, it seems obvious that a high amount of L-Carnitine is needed (above 3,000 mg) and that it must be administered separately from Orlistat, as according to this document combined administration of these active ingredients is not considered to obtain the weight loss side effect. Moreover, international application '07812 mentions that these ingredients may be present in a gelatine capsule with a division wall separating one active ingredient from the other, or a double-wall capsule, one inserted in the other with an independent active ingredient.

Another document discussing the use of L-Carnitine for the treatment of weight is the document by I.Lofgren, T.Zern, K.Herron, K.West, M.Sharman, J.Volek, N.Schachter, S.Koo, M.Fernandez, "Weight loss associated with reduced intake of carbohydrate reduces the atherogenicity of LDL in premenopausal women". Metabolism, Volume 54, Issue 9, Pages 1133-1141*,* which approaches a clinical trial aimed at evaluating the effect of intervention in three weight loss issues: a) diet changes (e.g., moderate energy restriction, carbohydrate reduction, protein increase), b) increase of physical activity, and c) use of L-Carnitine as diet supplement, particularly observing plasma lipids and atherogenicity of low-density lipoprotein particles in a population of premenopausal women with overweight and obesity. This was a double-blind, 10-week study in women aged 20 to 45 years. The treatment included diet with calorie restriction, increased physical activity and the intervention group was administered 3,000 mg/day of L-Carnitine. No significant weight loss was seen between the two groups.

Another document about L-Carnitine is that of *de* Derosa G, Cicero AG, Gaddi A, Mugellini A, Ciccarelli L, Fogari R. "The Effect of L-carnitine on plasma lipoprotein(a) levels in hypercholesterolemic patients with type 2 diabetes mellitus. Ciln Ther 2003; 25(5):1429-39*.* This document describes a double-blind, 26-week study including 47 men and 47 women aged from 47 to 57 years and with a BMI (Body Mass Index) of 27.3 ± 2.5 kg/m². A diet of 1,400 to 1,600 Kcal/day was prescribed in both groups. The intervention group received 2,000 mg/day of L-Carnitine. No significant weight loss or differences between the groups were seen at the end of the study.

Moreover, the document from Elmslie JL, Porter RJ, Joyce PR, Hunt PJ, Mann Jl. "Carnitine does not improve weight loss outcomes in valproate-treated bipolar patients consuming an energy-restricted, low-fat diet ". Bipolar Disord 2006;8:503-507 describes a study on the effect of L-Carnitine in 60 subjects with obesity (49 women and 11 men). A loss of 1.9 kg and 0.9 kg was seen in the treatment group (15 mg/kg/day of L-Carnitine) and the control group, respectively, assuming that patients of a weight of 70 kg need over 1050 mg per day of L-Carnitine. However, the weight loss seen is not significant and no differences were seen in the two groups. This study was performed over 26 weeks and included a restriction of 1,500 Kcal in diet.

From these three documents, it seems to be obvious and suggested that an amount over 1,000 mg of L-Carnitine is needed to achieve some weight loss. However, the weight loss is not always achieved or poor results are obtained.

On the other hand, discussing the management of the Orlistat compound, it shows sticking and poor flowability characteristics that make processing difficult; for this, complex techniques must be used to process it, one of them is mentioned in document WO/1998/034607 that describes the manufacture of Orlistat capsules from pellets or particles used a high shear blender granulator to mix Orlistat in a solvent, subsequently the mixture is run through an extruder, then through a pelletiser, and then the particles are placed in a fluidised bed and, finally, the particles are run through squared sieves where the particle size of the pellets obtained is selected, discarding those not meeting the proposed particle size.

The above is more important for manufacturing any pharmaceutical form from Orlistat, for instance capsules, as these are prepared from powder obtained by dry mixing, wet granulation or dry granulation methods. Therefore, an adequate method of preparation is required that gives Orlistat the appropriate physical characteristics, such as being a homogeneous powder, with free flowability to make its management easy when manufacturing a pharmaceutical form in large scale, for instance, capsules. Moreover, an innovative method is required for the efficient management of Orlistat if this is combined with another active ingredient.

### BRIEF DESCRIPTION OF THE INVENTION

Unexpectedly from that suggested and observed in the teachings of the prior art, during the development of this invention it was found that L-Carnitine and Orlistat may be used in combination in a pharmaceutical composition to reduce weight and, moreover, it was found that in the pharmaceutical composition of this invention it is not necessary to use amounts above 1,000 mg of L-Carnitine to reach this objective. The above is interpreted as a synergistic effect resulting of the combination of Orlistat with L-Carnitine, which results in a higher weight loss effect vs the administration of each of these compounds alone.

Another unexpected result is that only a single daily dose is needed to achieve a marked weight loss. Therefore, it is preferred to provide the pharmaceutical composition formulated in a single dose unit, preferably an oral dose unit.

In particular, the pharmaceutical composition to reduce weight in this invention is characterised by comprising a mixture of Orlistat and L-Carnitine as active ingredients or a pharmaceutically acceptable salt of each of them; and a pharmaceutically acceptable vehicle wherein the composition comprises 300 to 1,000 mg of L-Carnitine and 40 to 140 mg of Orlistat.

In an embodiment of the invention, Orlistat is adsorbed or coated by a diluent which allows for managing Orlistat adequately. For instance, it is preferred that this diluent is water-soluble, where this diluent is crystallised over Orlistat to coat it. And when the diluent is not alcohol-soluble, Orlistat is adsorbed over this diluent.

In an embodiment of the invention, the composition is formulated to be administered by oral route, preferably a pharmaceutical form in a capsule.

In another embodiment of the invention, a method is provided to manufacture the pharmaceutical composition as defined above; for this a technique was developed which allows for managing Orlistat easily and practically to easily mix it with L-Carnitine, avoiding the properties of Orlistat as a greasy, sticky ingredient.

More particularly, the method of this invention comprises the following steps: dry mixing Orlistat with a water-soluble diluent or a non-alcohol soluble diluent. Subsequently, the mixture is wetted and then granulated; then the granules of Orlistat obtained are dried; the dry granules of Orlistat are then sieved and then mixed with L-Carnitine granules and the pharmaceutically acceptable vehicle; after this step, optionally this composition its led to its final pharmaceutical formula, preferably a capsule.

In another embodiment of the invention a treatment method is provided for overweight or obesity, that consists of administration to a human patient of a therapeutically effective amount of the pharmaceutical composition of this invention as set out above and at the amounts specified, where administration is only once a day.

In an additional embodiment of the invention, it relates to the use of a pharmaceutical composition as defined above for manufacturing a medicinal product useful for the treatment of overweight or obesity, where the medicinal product is administered only once a day.

### BRIEF DESCRIPTION OF THE DRAWINGS

The novelties considered characteristic of this invention shall be established particularly in the attached claims. However, the invention itself, for both its ingredients, therapeutic action, obtaining method and other objects and advantages thereof, shall be better understood in the following detailed description of some preferred embodiments, when read related to the associated drawings, wherein:
Figure 1 shows the starting conditions of the patients involved in a first clinical trial, the patients had been divided into groups according to the specific treatment they received during the clinical trial.
Figure 2 shows the final conditions of the treatment groups of Figure 1, one of the groups had been treated with a preferred embodiment of the pharmaceutical composition of this invention.
Figure 3 shows the percentage of patients that at the end of the treatment returned to their normal weight in each group of the first clinical trial.
Figure 4 shows the percentage of patients that at the end of the treatment had their weight reduced, but were still rated as overweight in each group of the first clinical trial.
Figure 5 shows the percentage of patients that at the end of the treatment had their weight reduced but were still rated as grade 2 obesity in each group of the first clinical trial.
Figure 6 illustrates the average reduction in the body mass index in the patients in a second clinical trial that were treated with the composition of the invention herein.
Figure 7 shows the reduction in the body mass index achieved in the patients treated in the second clinical trial.

### DETAILED DESCRIPTION OF THE INVENTION

According to the present invention a pharmaceutical composition is provided that comprises a mixture of Orlistat and L-Carnitine as active ingredients or a pharmaceutically acceptable salt of each of them; and a pharmaceutically acceptable vehicle wherein the composition comprises 300 to 1,000 mg of L-Carnitine and 40 to 140 mg of Orlistat. The amounts used in the composition evidenced a marked effect on weight loss, above these limits an unnecessary amount would be supplied to reach the intended effect and below them an amount not achieving an effect on weight loss would be supplied.

In this description, the term pharmaceutically acceptable means a material compound or composition that within the scope of medical judgement are adequate for contact with human tissues, with no toxicity, irritation, allergic response or other excessive complications of concern according to a reasonable benefit/risk ratio.

The combination and mix of the active ingredients of the composition is more effective in weight loss as compared to their use alone. Therefore, the active ingredients have a synergistic effect due to their combination.

In a very specific embodiment, the composition comprises 600 mg of L-Carnitine, and in another additional embodiment it comprises 120 mg of Orlistat. These amounts are appropriate for manufacturing a pharmaceutical form of the composition with only one single daily dose to reduce weight. The composition is formulated more preferably in a single oral dosing unit.

The L-Carnitine salt used in the composition is L-Carnitine tartrate, though other salts may be used, such as levocarnitine, L-Carnitine fumarate, L-Carnitine orotate. In particular, L-Carnitine tartrate is preferred because it is the salt obtained more easily in the market and provides L-Carnitine at a higher amount vs other salts and is also more stable.

In a particular embodiment, it is preferred that in the pharmaceutical composition Orlistat is adsorbed or coated by a diluent which makes easier the method for obtaining the composition as set out below. More specifically, this diluent is water-soluble for Orlistat to be coated by this diluent, that is selected among the group comprising maltose, isomaltose, lactitol, sugar, mannitol, and dextrose.

In this case, when Orlistat is adsorbed over the diluent, this is diluent non-soluble in alcohol, for instance, an alcohol with 2 or 3 carbon atoms, such as ethyl alcohol or isopropyl alcohol. An example of diluent with these characteristics is calcium phosphate.

On the other hand, it is preferred that the pharmaceutical composition of this invention is formulate for oral administration, preferring the form of a capsule, a tablet, an oral solution, a powder for reconstitution adding a fluid, more preferably it is preferred to formulate the composition in a capsule, that may be a capsule made up of hard gelatine or soft gelatine, hydroxypropyl methylcellulose (HPMC) capsules and pululan (NP capsules). The capsules contains up to 15 % of the pharmaceutically acceptable vehicle to provide an adequate supply of the active ingredients.

Furthermore, with regard to the method for obtaining the pharmaceutical composition of this invention, first a dry mixing step is performed with Orlistat with a water-soluble diluent or a diluent non-soluble in alcohol.

Subsequently this mixture is wetted and granulated; then a granule drying step is performed over Orlistat to then perform sieving of these dry granules; Orlistat is dry mixed with granules of L-Carnitine and of the pharmaceutically acceptable vehicle. Finally, the mixture of these two actives ingredients is taken to the final pharmaceutical formula, preferably a capsule.

In a first preferred embodiment of the method of this invention, in the dry mixing step of Orlistat a water-soluble diluent is used, preferably lactose, maltose, isomaltose, lactitol, sugar, mannitol and dextrose; preferably this dry mixing step is performed in a high shear blender-granulator.

Then, in this first embodiment of the method, the wetting step is performed using preferably purified water to form wet granules that are subsequently sieved by a rotor mill. Subsequently, the step of drying the wet granule of Orlistat may be performed in a fluidised bed, that works preferably at room temperature. Once Orlistat granule is dry, sieving is performed using a rotor mill, where no joining or sticking problems are shown by Orlistat.

Subsequently, Orlistat granules are mixed with L-Carnitine and the pharmaceutically acceptable vehicle, that comprises preferably at least one water content adsorber or drier, such as magnesium metasilicate aluminium, talc and silicium dioxide, and a lubricant such as calcium stearate, zinc stearate, talc, sodium fumarate stearyl and magnesium stearate; this mixture of the active ingredients may be performed in a bin blender. Finally, the composition is encapsulated and no sticking problems caused by Orlistat are seen.

Specifically this first embodiment obtains granules of the pharmaceutical composition that are brittle, frail and not oily, which shows no problems during encapsulation. In particular, the wetting step with water of the mixture comprising Orlistat and the water-soluble diluent, e.g., lactose or the appropriate diluent, lactose solubilises and when it is dried causes crystallisation of lactose over Orlistat, which causes that the characteristic property of Orlistat as a greasy powder does not occur; consequently, the final mixing of Orlistat with L-Carnitine is enhanced, and, therefore, a capsule or other pharmaceutical forms of the composition in this invention can be manufactured more easily on a large scale basis.

In a second embodiment of the method of this invention, in the dry mixing step with Orlistat with the diluent, the latter is characterised in that it is a diluent not soluble in alcohol, of 2 or 3 carbon atoms, such as ethyl or isopropyl alcohol, the diluent may be calcium phosphate or others that are not soluble in these alcohols, mixing can be performed in a high shear blender, granulator.

Subsequently, during the wetting step of the above mixture, isopropyl alcohol or ethyl alcohol are used, forming granules, and then sieving of wet granules of Orlistat is performed using a rotor mill. Subsequently the wet granules are dried, preferably at room temperature using a fluidised bed

The dry granules of Orlistat are sieved using a rotor mill, with no joining or sticking problems. Then, final mixing of Orlistat with L-Carnitine and the pharmaceutically acceptable vehicle is performed, that comprises at least one water content adsorbent or drier and a lubricant; this final mixture can be obtained using a bin blender. Finally, the composition is led to its final pharmaceutical formula by encapsulation with no sticking problems caused by Orlistat.

In this second embodiment, where the diluent is not alcohol-soluble, upon adding isopropyl alcohol during the wetting step, Orlistat is solubilised in the alcohol and is adsorbed over calcium phosphate or the respective diluent not soluble in alcohol. When drying this mixture, the granule of Orlistat formed has the appearance of calcium phosphate, that is known to be easily sieved and shows no sticking problems during the subsequent method steps.

Reviewing both embodiments of the method overall, the embodiment using a water-soluble diluent shows the advantage of being an aqueous method with no exposure to solvents, while in the embodiment of the diluent not soluble in alcohol, the density of the pharmaceutical composition may be higher and allow for capsule filling, for instance a capsule of size 00 elongated (capacity of 1.02 millilitres). However, it must be noted that both embodiments are useful to obtain the composition of this invention.

In the method of this invention, where wet granulation of Orlistat is performed, it is important that wetting of the components with the wetting agent that may be water or alcohol ensures that the components acquire an adequate consistency for obtaining the formation of uniform granules of Orlistat by sieving and drying. The final wetting point is achieved when taking part of the sample with the hand and pressing it gently provides a firm mass that broken to a half is divided into two portions and is not fully fragmented. In this event, wet sieving can be performed for obtaining uniform granules of Orlistat.

During the wet granule drying step over Orlistat, an attempt is made to obtain homogeneous water content and it is advisable to maintain a residual water content amount, so that the different components remain in a hydrated state; therefore, controls should be performed on the water content of granules until the residual water content specified for the composition is obtained. Temperature control is important to ensure stability of the components subjected to granulation. Preferably the drying temperature is 35.0 °C to 40.0 °C and the residual water content of granules must not exceed 1.0 %.

During the sieving step of the composition, it is intended to perform the reduction and standardisation of particle size (comminution) using stainless steel meshes to obtain finely divided powders with a controlled particle size. This is performed providing the powder with an adequate fluidity and compactation.

Finally, it is also important that the particle size of the ingredients and vehicles and diluents used in the composition is uniform, because the particles of different sizes tend to stratify at rest, with motion or during transport, which results in an inaccurate filling of the active ingredients. For this purpose, in the case of active ingredients and vehicles (except for magnesium stearate) they are run through final mesh 24, which gives a particle size of about 701 microns. This particle size provides the necessary rheological characteristics to form for instance a capsule with the composition. In the case of magnesium stearate, this is run through mesh 60 to create a smaller particle size and a greater surface area than the rest of the formulation, thus enhancing product lubrication.

Another important factor to be considered in the method is that since the mixtures of both active ingredients performed sequentially have a more homogeneous distribution of its components than the mixtures obtained at random, care must be taken with the order of the ingredients, also verifying complete homogenisation before adding the next component. Furthermore, as mixing times are established and repeated in each batch, this ensures the standardisation of the process among the different product batches.

This control is required to prevent segregation or changes in the active ingredient content in the product. Mixing times and orders of addition of starting materials are verified according to the manufacturing method.

It is important that the relative humidity is within the specifications to prevent that this environmental factor influences the rheological behaviour of the product thus affecting the encapsulation process. Therefore, the periodic verification throughout the process, and it must not exceed 60 % of the relative humidity, above this limit the higher relative humidity in the composition may be affected functionally from its flow or assessment; therefore dewetting measures should be adopted in case the established specifications are exceeded.

The composition of this invention is useful for the treatment of overweight and obesity in a human patient; for this, the composition is supplied at a therapeutically effective amount of the active ingredients, as set out above and only once a day, which is extremely practical, as patients prefer a single dose instead of multiple daily doses.

The pharmaceutical composition for weight reduction and the method for obtaining it shall be more clearly illustrated by means of the examples described below, which are provided only by way of illustration, but not limiting it, and are namely:

### EXAMPLE 1

### Compatibility between the active ingredients and the vehicle

For the purpose of establishing the physicochemical behaviour between the two active ingredients, as well as the active ingredients and the vehicle, the following tests were performed,:
**1.1 Active agent - active agent compatibility:** independent samples were prepared of both L-Carnitine tartrate, Orlistat, and the binary mixture of L-Carnitine tartrate and Orlistat according to the percentages required in the composition. These samples were placed in clear type I glass vial bottles with chlorobutyl stopper and aluminium recap and were exposed to a temperature of 40 °C for 21 days; after this period, the results did not show evidence of degradation or incompatibility between the active ingredients under the test conditions.
**1.2 Active agent - vehicle compatibility:** ternary samples of L-Carnitine + Orlistat - Vehicle were prepared according to the concentrations required in the composition. The samples were placed in clear type I glass vial bottles with chlorobutyl stopper and aluminium recap and exposed to a temperature of 40 °C for 21 days; during this time no evidence of degradation or incompatibility between the components of the mixtures were seen under the test conditions.
Tables 1 to 7, 7A and 7B show specific embodiments of the composition of this invention that may be prepared.

**Table 1**

| **Component** | **Amount** |
|---|---|
| L-Carnitine tartrate | 0.6000 g |
| Orlistat | 0.0600 g |
| Lactose mesh 100 | 0.0500 g |
| Gasil gm2f | 0.0400 g |
| Micronised sodium lauryl sulphate | 0.0100 g |
| Magnesium stearate | 0.0100 g |
| Purified water | 0.0335 ml |
| Elongated pink hard gelatine capsule No. 00 | 1.0000 pz |

**Table 2**

| **Component** | **Amount** |
|---|---|
| L-Carnitine tartrate | 0.60000 g |
| Orlistat | 0.06000 g |
| Refined gum lacquer | 0.03333 g |
| Sodium starch glycolate | 0.02000 g |
| Magnesium stearate | 0.018000 g |
| 96° ethyl alcohol ** | 0.07900 ml |
| Elongated hard gelatine capsule No. 00 | 1.0000 pz |

**Table 3**

| **Component** | **Amount** |
|---|---|
| L-Carnitine tartrate | 0.6000 g |
| Orlistat, | 0.1200 g |
| Lactose mesh 100 | 0.1000 g |
| Gasil gm2f | 0.0400 g |
| Micronised sodium lauryl sulphate | 0.0090 g |
| Magnesium stearate | 0.0090 g |
| Purified water | 0.0670 ml |
| Elongated hard gelatine capsule No. 00 | 1.0000 pz |

**Table 4**

| **Component** | **Amount** |
|---|---|
| L-Carnitine tartrate | 0.6000 g |
| Orlistat | 0.1200 g |
| Gasil gm2f | 0.0300 g |
| Isopropyl alcohol | 0.02 ml |
| Sodium fumarate stearyl | 0.0040 g |
| Elongated hard gelatine capsule No. 00 | 1.0000 pz |

**Table 5**

| **Component** | **Amount** |
|---|---|
| L-Carnitine tartrate | 0.6000 g |
| Orlistat | 0.1200g |
| Refined gum lacquer | 0.03333 g |
| Sodium starch glycolate | 0.02100 g |
| Magnesium stearate | 0.01900 g |
| 96° ethyl alcohol | 0.08100 ml |
| Elongated hard gelatine capsule no. 00 | 1.0000 pz |

**Table 6**

| **Component** | **Amount** | **% w/w** |
|---|---|---|
| L-Carnitine tartrate | 600 mg | 69.77 % |
| Orlistat | 120 mg | 13.95 % |
| Granular tricalcium phosphate | 100 mg | 11.63 % |
| Silicon dioxide NF | 30 mg | 3.49 % |
| Magnesium stearate | 10 mg | 1.16 % |

**Table 7**

| **Component** | **Amount** | **% w/w** |
|---|---|---|
| L-Carnitine tartrate | 600 mg | 78.02 % |
| Orlistat | 60 mg | 7.80 % |
| Monohydrated lactose mesh 100 | 50 mg | 6.50% |
| Silicon dioxide NF | 40 mg | 5.20 % |
| Mic. Sodium lauryl sulphate | 9 mg | 1.17 % |
| Magnesium stearate | 10 mg | 1.30 % |

**Table 7A**

| SOFT GELATINE | | |
|---|---|---|
| **Description** | **Each capsule contains:** | % |
| L-Carnitine tartrate | 0,600 g | 54.55 % |
| Orlistat | 0,120 g | 10.91 % |
| Geloil | 0,127 g | 11.55% |
| Maize starch | 0,173 g | 15.73 % |
| Soy lecithin | 0,023 g | 2.09 % |
| Capryol PGMC | 0,050 g | 4.55 % |
| Simethicone Q7-2243 | 0,007 g | 0.64 % |

**Table 7B**

| SOFT GELATINE | | |
|---|---|---|
| Description | Each capsule contains: | % |
| L-Carnitine tartrate | 0,600 g | 60.00 % |
| Orlistat | 0,060 g | 6.00 % |
| Geloil | 0,127 g | 12.70 % |
| Maize starch | 0,155 g | 15.50 % |
| Soy lecithin | 0,025 g | 2.50% |
| Capryol PGMC | 0,025 % | 2.50 % |
| Simethicone Q7-2243 | 0,007 g | 0.70 % |

| | | |
|---|---|---|
| Commercial mixture of hydrogenated soy oil, GELOIL: glyceryl distearate and polyglyceryl-3-dioleate CAPRYOL PGMC: Propylene glycol caprilate SIMETHICONE Q7-2243: Low-density simethicone | | |

### EXAMPLE 2

### Method to obtain the pharmaceutical composition using a water-soluble diluent

A pharmaceutical composition was prepared as a capsule in compliance with the ingredients and amounts set out in Table 8.

**Table 8**

| **Ingredient** | **Per capsule** | **% w/w** |
|---|---|---|
| L-Carnitine tartrate | 600 mg | 78.02 % |
| Orlistat | 60 mg | 7.80 % |
| Monohydrated lactose mesh 100 | 50 mg | 6.50% |
| Silicon dioxide NF | 40 mg | 5.20 % |
| Micronized sodium lauryl sulphate | 9 mg | 1.17 % |
| Magnesium stearate | 10 mg | 1.30% |

Specifically, 100 % of the amount of Orlistat and 100 % of the amount of lactose mesh 100 were mixed in a high shear blender-granulator for 2 minutes and working at 52 rpm. The mixture was subsequently wetted with purified water and granulated. Then the wetted mixture was sieved through a 8 mm screen for forming granules of homogeneous size. Subsequently, drying was performed to a final water content established for the semi-finished product (not greater than 0.2 %). The total granules were sieved in a rotor mill to make particle size homogeneous and were added to the blender. Then silicon dioxide, micronised sodium lauryl sulphate and the total L-Carnitine tartrate, previously sieved, were added. They were mixed for 8 min at 10 rpm, adding magnesium stearate and mixing for 3 minutes at 20 rpm. Subsequently this mixture was encapsulated in elongated capsules of size no. 00.

### EXAMPLE 3.

### Method for obtaining the pharmaceutical composition using a diluent not soluble in alcohol

**Table 9**

| **Ingredient** | **Per capsule** | **%w/w** |
|---|---|---|
| L-Carnitine tartrate | 600 mg | 69.77 % |
| Orlistat | 120 mg | 13.95 % |
| Granular tricalcium phosphate | 100 mg | 11.63 % |
| Silicon dioxide NF | 30 mg | 3.49 % |
| Magnesium stearate | 10 mg | 1.16 % |

Specifically, 100 % of the amount of Orlistat and 100 % of the amount granular tricalcium phosphate were mixed in a high shear blender-granulator for 2 minutes and working at 52 rpm. The mixture was subsequently wetted with isopropyl alcohol and granulated. Then the wetted mixture was sieved through a 8 mm screen for forming granules of homogeneous size. Subsequently, drying was performed to a final water content established for the semi-finished product (not greater than 0.2 %). The total granules were sieved in a rotor mill to make particle size homogeneous and were added to the blender. Then silicon dioxide nf, and the total L-Carnitine tartrate, previously sieved, were added. They were mixed for 7 min at 10 rpm, adding magnesium stearate and mixing for 3 minutes at 20 rpm. Subsequently this mixture was encapsulated in elongated capsules of size no. 00.

### EXAMPLE 4

### A comparative, open-label, controlled clinical trial to evaluate the efficacy and safety of the composition Orlistat/L-Carnitine (OL-CA) vs Orlistat (OL) and L-Carnitine (CA) in adults with overweight above 10 %

For the purpose of demonstrating that a pharmaceutical composition comprising as active ingredients Orlistat 120 mg and Carnitine 600 mg associated with diet and exercise is more effective than the active ingredients alone Orlistat (OL) and L-Carnitine (CA) in weight reduction administered by oral route, a clinical trial was performed with the following characteristics.

### Study design

A randomised, parallel, cross-over, prospective, open-label clinical trial.

### Total sample size

60 patients.

### Study population

Male and/or female subjects aged above 18 years, with overweight above 10 % their ideal weight.

### Doses of the composition

- Group 1. Orlistat (OL) 120 mg, one capsule a day for 12 weeks.
- Group 2. L-Carnitine (CA) 600 mg, one capsule a day for 12 weeks.
- Group 3. Orlistat 120 mg and L-Carnitine 600 mg (OL-CA) in one capsule a day for 12 weeks.

### Other treatments

- No other drug therapy was permitted for obesity and overweigh using any administration route during the clinical trial.

### Distribution

- Three treatment groups with random distribution 1:!;1

### Inclusion criteria

- Male and/or female subjects over 18 years of age.
- Overweight above 10 % of their ideal weight or a Body Mass Index (BMI) of 28 kg/m2 or higher.
- Poor response to previous diet therapy.
- No participation in a clinical trial with a test drug or device for 30 days before the planned start of treatment.
- Patients with or without risk factors associated with obesity (type 2 diabetes and systemic hypertension).
- Following a nutritionally balanced and moderately hypocaloric plan containing about 30 % of calories from fat.
- Women with child-bearing potential should use some contraceptive method during the treatment.

### Exclusion criteria

- Under 18 years of age
- History of hypersensitivity to the components of the formula
- Patients with any organ failure
- Patients with a history of intolerance to lactose
- Patients with a history of bowel malabsorption syndrome and cholestasis
- Patients with suspected or diagnosed pancreatic disorder
- Pregnant and/or nursing women
- Diagnosed or suspected thyroid disorder
- Patients currently on anticoagulants or cyclosporine and amiodarone.

### Results

Randomisation in to the study was stratified at a 1:1:1 ratio. The subjects were classified according to their body mass index.
Group A, 20 subjects with overweight, BMI of 25 to 29.9 kg/m2
Group B, 20 subjects with grade 2 obesity, BMI of 30-39 kg/m2
Group C, 20 subjects with grade 3 obesity BMI above 40 kg/m2
With regard to the BMI, the following values and interpretation are given
- BMI 20 to 25 kg/m² = normal weight
- BMI 25 to 29.9 kg/m² = Overweight
- BMI 30 to 39 kg/m² - Grade 2 obesity
- BMI above 40 kg/m² = Grade 3 obesity

Of the 60 subjects enrolled, the most prevalent was the age group between 18 and 30 years, 45.0 %; the group at 31 to 40 years of age, 25.0 %; 41 to 50 years, 15.0 %; 51 to 60 years, 8.3 %, and the group of 61 to 70 years, 6.6 %.

With regard to distribution by study group, the female sex prevailed, with 46.6 %, and the predominant study group had grade 2 obesity (BMI 30 to 39 kg/m²).

### Evaluation

Of the subjects enrolled, the treatment groups were formed as follows:
**Group OL+CA** (Orlistat + L-Carnitine) 20 subjects; of whom 15 % (3 subjects n = 20) belonged to the overweight group, 45 % (9 subjects n = 20) to subjects with grade 2 obesity, and 40 % (8 subjects n = 20) grade 3 obesity.
**Group OL** (Orlistat alone) 20 subjects, of whom 5 % (1 subject n = 20) belonged to the overweight group, 60 % (12 subjects n = 20) with obesity 2, and 35 % (7 subjects n = 20) with grade 3 obesity.
**Group CA** (L-Carnitine alone) 20 subjects, of whom 10 % (2 subjects n = 20) belonged to the overweight group, 50 % (10 subjects n = 20) to subjects with grade 2 obesity and 40 % (8 subjects n = 20) to subjects with grade 3 obesity.

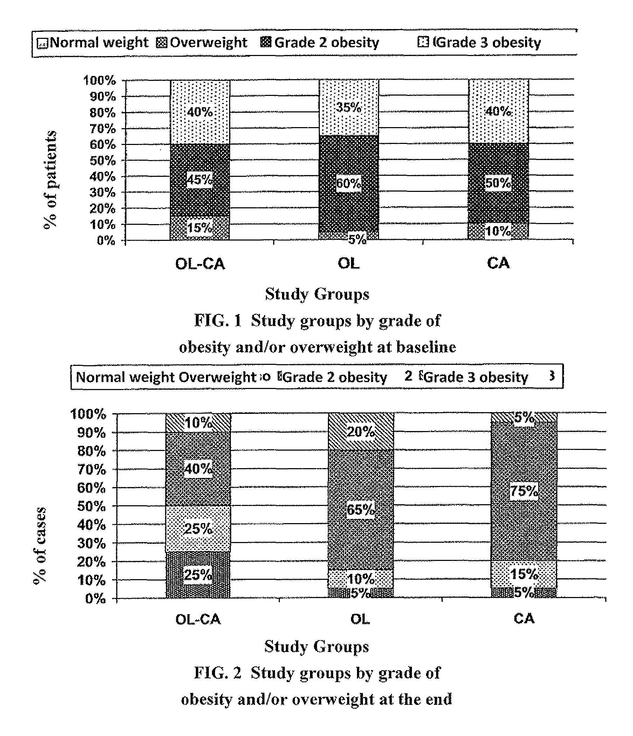

Figures 1 and 2 shows the data at the start and end of treatment.

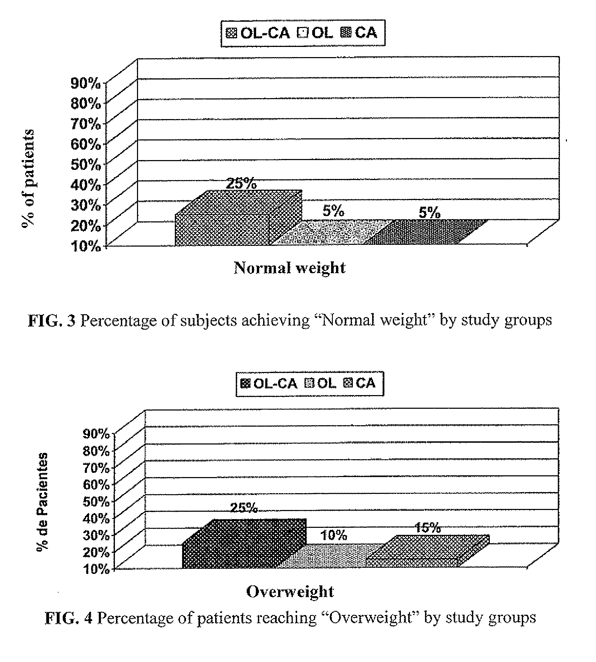

At the end of treatment (12 weeks) in the OL-CA group 100 % of the subjects completed the study, of whom 25 % (5 subjects n = 20) reached a normal weight, as compared to the OL and CA groups, where only one subject (5 %) in each group documented normal weight, see Figure 3.

With regard to subjects with overweight, in the OL-CA group 25 % (5 subjects) documented overweight as compared to the control groups, where the OL group documented 2 subjects (10 %) and in the CA group 3 subjects (15 %), see Figure 4.

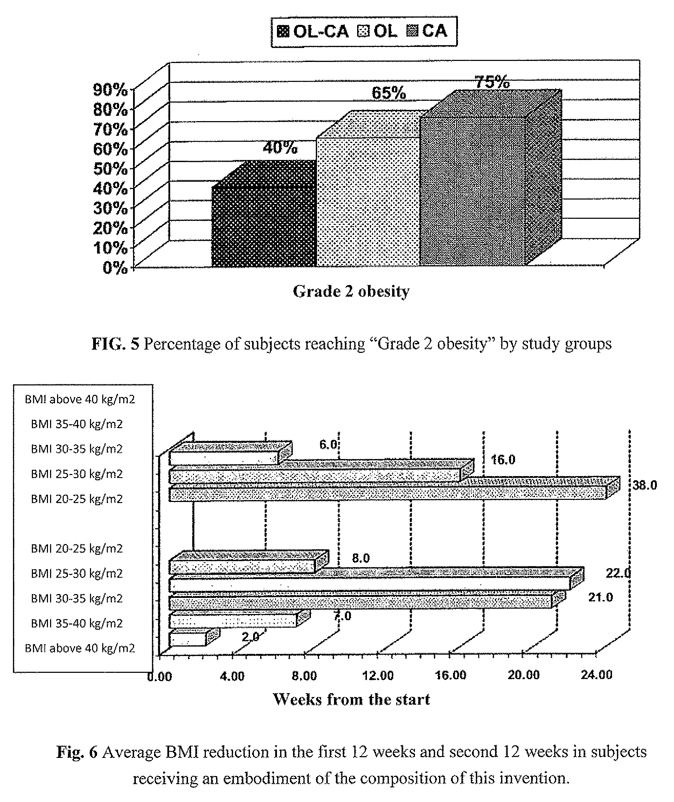

With regard to the patients reaching grade 2 obesity, the OL-CA group was 40 % (8 subjects n = 20), in the OL group 65 % (13 subjects n = 20) and in the CA group 75& (15 subjects n = 20), see Figure 5.

Finally, with regard to the subjects with grade 3 obesity in the OL-CA group it was documented 10 % (2 subjects n = 2), OL 20 % (4 subjects n = 20), and in the CA group 5 % (1 subject).

With regard to tolerability the evaluation was excellent in 80 % (48/60) of the subjects enrolled, very good in 15 % (9/60) Good in 5 % (3/60).

The average monthly weight loss was 2.4 + 2.3 kg, the results show statistically significant differences, the average weight at the end of the study was above 10 % in the OL-CA group, non-significant weight losses were documented in the control groups.

The combination Orlistat + L-Carnitine caused a higher weight loss, with efficacy, safety and very few adverse events as compared to the other study groups.

### EXAMPLE 5

### Clinical trial to evaluate the efficacy and safety of the composition of Orlistat + L-Carnitine (O+LC) for the treatment of adolescents with obesity and/or overweight

The study objective was to demonstrate the clinical efficacy of the composition of this invention dosing it once a day, in weight loss of 5 % or more in the first 12 weeks of treatment and a weight loss of 10 % in the second 12 weeks.

### Secondary objective:

- To evaluate the safety and quality of life of the patients.
- To evaluate the tolerability of the combination.
- To evaluate treatment response

### Study design

- A prospective, single-blind study.

### Study population

- Male and/or female subjects aged from 12 to 16 years with BMI of 2 kg/m² or higher than the average weight for the percentile 95 based on age and sex.

### Dosing the composition

- Oral capsules, one capsule every 24 hours, containing 60 mg of Orlistat and 600 mg of L-Carnitine tartrate.

### Other treatments

- No other treatment is permitted with another drug therapy for obesity and overweight by any administration route during the clinical trial.

### Duration of treatment

- 24 weeks. It was a prospective, single-blind clinical trial comprising 7 stages/steps: Assessment (1), Evaluation (2,3,4,5,6,) and end of treatment visit (7).
- The number of subjects enrolled was 60 patients, with the following percentages: 38 female patients 63.33 % (n = 38) and 22 male subjects 36.66 % (n = 22) (Table 10); the average age was 13.3 years ± 1.6 years.

**Table 10**

| | Female | | Male | | Total | |
|---|---|---|---|---|---|---|
| **Age group** | **No. of patients** | **%** | **No. of patients** | **%** | **No. of patients** | **%** |
| **12-13 years** | **12** | **31.6** % | **8** | **36.4 %** | **20** | **33.3 %** |
| **13-14 years** | **8** | **21.1 %** | **6** | **27.3 %** | **14** | **23.3 %** |
| **14-15 years** | **8** | **21.1 %** | **5** | **22.7 %** | **13** | **21.7 %** |
| **15-16 years** | **10** | **26.3 %** | **3** | **13.6 %** | **13** | **21.7 %** |
| **Total** | **38** | **100.0 %** | **22** | **100.0 %** | **60** | **100.0 %** |

| | | | | | | |
|---|---|---|---|---|---|---|
| χ² = 7,504; p = 0.277; N.S. | | | | | | |

All subjects enrolled had obesity and/or overweight according to the body mass index (Table 11).

**Table 11**

| | | |
|---|---|---|
| **BMI baseline visit** | **No. of patients** | **%** |
| **30-35 kg/m2** | 38 | 63.3 % |
| **35-40 kg/m2** | 16 | 26.7 % |
| **Above 40 kg/m2** | 6 | 10.0 % |
| **Total patients** | 60 | 100.0 % |

The average general weight per age and sex was 84.1 kg ± 1.1 kg for the age group of 12 to 13 years, 70 ± 1.3 kg for the group of 13 to 14 years 80.2 ± 1.5 kg for the group of 14 to 15 years 90.5 ± 1.1 kg and for the group of 15 to 16 years 100.7 ⁺ 1.6 kg (Table 5), the average height was 172 + 15 cm (Table 12).

**Table 12**

| Table 12 Average weight of 60 patients per age and sex, receiving a composition of L-Carnitine and Orlistat for the treatment of obesity and overweight | | | | | | |
|---|---|---|---|---|---|---|
| Baseline weight visit | Age | Fem no. | | Male no. | No. of pats. | % |
| 60-70 kg | 12-13 years | 7 | | 9 | 16 | 26.6 % |
| 70-80 kg | 13-14 years | 6 | | 4 | 10 | 16.6 % |
| 80-90 kg | 14-15 years | 13 | | 15 | 28 | 46.6 % |
| 90-100 kg | 15-16 years | 2 | | 4 | 6 | 10. % |
| TOTAL | | 28 | 3 | 32 | 60 | 99.8 % |

94 % of the subjects enrolled performed physical activity for about 1 hr a day (sports at their schools), the remaining 6 % performed an additional physical activity at least twice a week, besides their school sports.

As a relevant history, 98.2 % of the subjects enrolled mentioned that they played about 3 to 4 hours a day, including the weeks, some type of videogame.

### Evaluation

The 60 subjects completed the end of treatment visit, five adverse events were reported, in 3 subjects (5.0 %) with colic type abdominal pain, administering as single dose butylhyoscine without requiring another type of concomitant therapy and without exclusion from the study. One subject (1.6 %) reported diarrhoeal stools, that did not required concomitant therapy, and one subject (1.6 %) reported constipation that required diet changes with an increase in fibres and fluid, not needing the use of drug therapy (Table 13).

**Table 13**

| Table 13 Adverse events reported in 5 patients receiving a composition of L-Carnitine and Orlistat | | | |
|---|---|---|---|
| **Adverse event** | **Female** | **Male** | **No. of patients** |
| Abdominal pain | 2 | 1 | 3 |
| Diarrhoea | 1 | 0 | 1 |
| Constipation | 0 | 1 | 1 |
| | | | |
| **Total** | 3 | 2 | 5 |

BMI decreased an average of 5.3 % in the first 12 weeks, in the second twelve weeks the BMI reduction reached as average was statistically significant (see Figure 6).

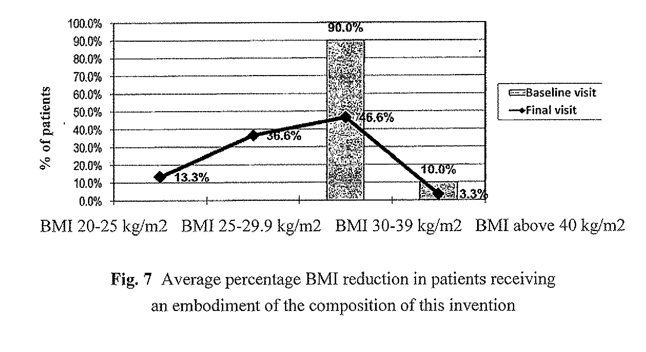

The weight loss persisted, and eight subjects, 13.3 %, could reach their normal weight; 36.6 % (22) documented overweight; 46.6 % (28) grade 2 obesity, and only 3.3 % (2) grade 3 obesity as compared to the baseline visit, where 90.0 % (54 subjects) had grade 3 obesity and the remaining 10 % (6 subjects) reported grade 3 obesity (Figure 7).

The Orlistat + L-Carnitine composition was shown to be effective and safe for the treatment of weight loss in adolescents with overweight and grade 2 or 3 obesity.

Even though preferred embodiments of this invention have been described and exemplified, it must be noted that multiple changes can be made on them, such as the final pharmaceutical forms of the compositions, the selection of the salt of the active ingredients, etc. Therefore, this invention should not be considered as restricted except as required by the prior art and for the scope of the attached claims.

## Claims

1. A pharmaceutical composition for reducing weight, **characterised by** comprising a mixture of Orlistat and L-Carnitine as active ingredients or a pharmaceutically acceptable salt of each one of them; and a pharmaceutically acceptable vehicle, where the composition comprises 300 to 1,000 mg of L-Carnitine and 40 to 140 mg of Orlistat.

2. The pharmaceutical composition according to claim 1, further **characterised in that** L-Carnitine salt is selected from the group comprising L-Carnitine tartrate, levocarnitine, L-Carnitine fumarate, or L-Carnitine orotate.

3. The pharmaceutical composition according to claim 2, further **characterised in that** L-Carnitine salt is L-Carnitine tartrate.

4. The pharmaceutical composition according to any of claims 1 to 3, further **characterised in that** Orlistat is adsorbed or coated by a diluent.

5. The pharmaceutical composition according to claim 4, further **characterised in that** this diluent is water-soluble, wherein the diluent is crystallised over Orlistat to coat it..

6. The pharmaceutical composition according to claim 5, further **characterised in that** this water-soluble diluent is selected from the group comprising maltose, isomaltose, lactitol, sugar, mannitol, and dextrose.

7. The pharmaceutical composition according to claim 4, further **characterised in that** Orlistat is adsorbed over the diluent.

8. The pharmaceutical composition according to claim 7, further **characterised in that** the diluent is not alcohol-soluble.

9. The pharmaceutical composition according to claim 8, further **characterised in that** the diluent is not soluble in an alcohol of 2 or 3 carbon atoms.

10. The pharmaceutical composition according to claim 9, further **characterised in that** the diluent is calcium phosphate.

11. The pharmaceutical composition according to any of claims 1 to 10, further **characterised in that** the composition comprises 600 mg of L-Carnitine.

12. The pharmaceutical composition according to any of claims 1 or 11, further **characterised in that** the composition comprises 120 mg of Orlistat.

13. The pharmaceutical composition according to any of claims 1 to 12, further **characterised in that** the composition is formulated as to be orally administered.

14. The pharmaceutical composition according to claim 13, further **characterised in that** it is formulated in a single oral dose.

15. The pharmaceutical composition for reducing weight, according to claim 13 or 14, further **characterised in that** the composition is formulated as a capsule, a tablet, or a powder for reconstitution adding a fluid.

16. The pharmaceutical composition for reducing weight, according to claim 15, further **characterised in that** the composition is formulated as a capsule.

17. The pharmaceutical composition for reducing weight, according to claim 16, further **characterised in that** the capsule is made up of hard gelatine or soft gelatine.

18. The pharmaceutical composition for reducing weight, according to claims 1 to 17, useful for the treatment of obesity and overweight.

19. A method for manufacturing a pharmaceutical composition according to any of claims 1 to 18, the method **characterised by** comprising the following steps:
a) dry mixing granules of Orlistat with a water-soluble diluent or a diluent not soluble in alcohol;
b) wetting the mixture of step a) to obtain wet granules of Orlistat;
c) dry the wet granules of Orlistat;
d) sieve the dry granules of Orlistat, and
e) dry mix the granules of Orlistat with granules of L-Carnitine and the pharmaceutically acceptable vehicle.

20. The method according to claim 19, further **characterised in that** in step a) the diluent is water-soluble.

21. The method according to claim 20, further **characterised in that** the diluent is selected from the group comprising maltose, isomaltose, lactitol, sugar, mannitol, and dextrose.

22. The method according to claim 19, further **characterised in that** in step a) the pharmaceutically acceptable diluent is not soluble in an alcohol of 2 to 3 carbon atoms.

23. The method according to claim 22, further **characterised in that** the pharmaceutically acceptable diluent not soluble in alcohol is calcium phosphate.

24. The method according to any of claims 19 to 23, further **characterised in that** in step d) the pharmaceutically acceptable vehicle comprises at least a humidity adsorbent and a lubricant.

25. The method according to any of claims 19 to 24, further **characterised in that** after step e) a step of composition encapsulation is carried out.

26. The use of a pharmaceutical compositions according to any of claims 1 to 18 for manufacturing a medicinal product useful in the treatment of obesity and overweight.
